# EUROPEAN PATENT APPLICATION

(11) **EP 4 632 061 A1**
(43) Date of publication of application: **15.10.2025**
(21) Application number: 25165285.5
(22) Date of filing: 21.03.2025
(51) Int. Cl.: C12N 1/06, C12N 1/12, C12N 13/00, C12P 7/6463

(54) **BIOTECHNOLOGICAL PROCESS FOR OBTAINING COMPONENTS FROM MICROORGANISMS WITH A CELL WALL**

(30) Priority: 21.03.2024 DE 102024108146
(71) Applicant: Prevo Biotech UG (haftungsbeschränkt), 85354 Freising (DE)
(72) Inventor: KADDOURA, Khalil, 85354 Freising (DE)
(74) Representative: Winter, Brandl - Partnerschaft mbB

(57) **Abstract**

The invention relates to a biotechnological process for obtaining components from microorganisms with a cell wall, comprising a process step selected from the group consisting of a bead milling step, a high-pressure homogenization step, an ultrasound treatment step or a combination thereof (step a)), an enzymatic treatment step (step b)), and optionally a cytolysis step (step c)).

The invention further relates to the use of the components obtained by the biotechnological process as nutrient composition, food product, food supplement, foodstuffs, food ingredients, pharmaceutical ingredients, feed ingredients, cosmetics, biofertilizer, soil additive or culture medium for cell cultures.

## Description

### TECHNICAL FIELD OF THE INVENTION

The present invention relates to a biotechnological process for obtaining components from microorganisms with a cell wall and to the use of the components obtained by the biotechnological process as a nutrient composition, food product, food supplement, foodstuffs, food ingredients, pharmaceutical ingredients, feed ingredients, cosmetics, biofertilizer, soil additive or culture medium for cell cultures.

### BACKGROUND OF THE INVENTION

Microorganisms are microscopically small, mostly single-celled organisms.

Microorganisms include microalgae such as chlorella, bacteria such as lactic acid bacteria or cyanobacteria, and fungi such as baker's yeast. Many microorganisms, including those mentioned here, have a cell wall that lies outside the cell membrane and whose main function is to prevent osmotic lysis of the cell.

Microorganisms fulfil a wide range of tasks. They play an important role in the human body. For example, bacteria form the intestinal flora in the gastrointestinal tract and thereby prevent, among other things, the colonisation and spread of pathogenic bacteria and fungi.

They metabolise chemical elements such as carbon and nitrogen and produce at least half of the Earth's elemental oxygen. They play a central role in geochemical cycles such as the nitrogen cycle.

Microorganisms also represent an alternative to toxic chemical agents in pest control and are increasingly used in energy production or the biodegradation of waste and pollutants.

One example of this is oil spills at sea, which are caused, for example, by oil tanker accidents. Special microorganisms can absorb the pollutants from the sea.

Some microorganisms are already produced industrially and serve as a valuable source of a variety of substances, materials and metabolites such as fatty acids, proteins, sugars and vitamins, pigments, biomass for aquaculture and for wastewater treatment.

In biotechnology, microorganisms are used to produce pharmaceuticals such as antibiotics and insulin or technically usable substances such as oils or fuels.

Microorganisms can be cultivated in basins, tanks, photobioreactors and fermenters using different techniques and volumes depending on the species cultivated and the respective applications.

The advantages of using microorganisms for industrial purposes lie in their high growth and proliferation rate in nutrient-rich water. In addition, they can grow in very warm climates and in salt water without consuming fresh water resources and can be cultivated on much smaller areas than is the case in conventional agriculture, for example.

Microorganisms also produce a homogenous biomass that is not divided into components with different properties, as is the case with terrestrial plants with seeds, fruits, leaves, stems and roots.

Conventional industrial processes for obtaining substances, materials and metabolites from cell-wall-containing microorganisms often achieve only a low yield of native substances, materials and metabolites because they use rough mechanical processes or aggressive chemicals to break down the cell wall and/or membrane.

EP 0906414 B2 relates to a process for producing microbial biomass and obtaining components therefrom by granulating the biomass and extruding it to extract the desired components without disrupting the cells.

EP 2819507 B1 relates to a process for extracting molecules from algae using solvents and high pressures and temperatures.

However, there is still a need to develop a biotechnological process by which proteins, vitamins, fats, sugars or a combination thereof can be obtained from cell-wall-comprising microorganisms for use as a nutritional composition, a food product, a food supplement, foodstuffs, food ingredients, pharmaceutical ingredients or a culture medium for cell cultures.

There is a particular need for the development of a simple and gentle, sustainable and cost-effective process by which proteins, vitamins, fats, sugars or a combination thereof can be obtained step by step in high yields and specifically from microorganisms that comprise cell walls.

The present invention aims to provide a solution for the increasing demand for inexpensive and gentle industrial biotechnological processes to obtain proteins, vitamins, fats, sugars or a combination thereof specifically, in a native state and in high yields from microorganisms with cell walls. These can be used as nutrient compositions, food products, food supplements, foodstuffs, food ingredients, pharmaceutical ingredients or culture media for cell cultures.

### OBJECT OF THE INVENTION

The object of the present invention is to provide interstitial compounds and extracellular compounds, sugars, oligosaccharides, fibers, vitamins, antioxidants, minerals and enzymes, proteins, fats and/or oils, carotenoids, a sludge containing spent biomass, vitamins or combinations thereof natively, in high yield and specifically from cell wall-comprising microorganisms, which can be used a nutrient composition, food product, food supplement, foodstuffs, food ingredients, pharmaceutical ingredients, feed ingredients, cosmetics, biofertilizer, soil additive or culture medium for cell cultures.

This task is solved with the help of the independent claims. The dependent claims further develop the core idea of the invention.

### SUMMARY OF THE INVENTION

**In a first aspect,** the invention provides for a biotechnological process for obtaining components from microorganisms with a cell wall, which comprises the following process steps: a) a process step selected from the group consisting of a bead milling step, a high-pressure homogenization step, an ultrasound treatment step or a combination thereof, b) an enzymatic treatment step, and optionally c) a cytolysis step.

Further steps may be included between steps a), b) and c).

Preferably, step a) serves to dissolve the cell wall, more preferably step a) serves to cavitate the outer cell wall layer, which is composed of insoluble complex polysaccharides and results in the exposure of the components of the inner cell wall layer material.

Figure 1 shows a characteristic structure of the cell wall of microalgae as an example of a microorganism with a cell wall. The cell wall includes the outer cell wall layer and the inner cell wall layer.

Figure 2 shows the process steps and their sequence. Microalgae are provided as an example of the starting material being microorganisms with a cell wall.

Before step a), fermentation media components such as glucose and minerals are removed, which both might impact the performance of the biotechnological process. Consequently, extracellular and interstitial compounds are enriched.

In process a), the bead milling step, the high-pressure homogenization step, and the ultrasound treatment step or a combination thereof are performed as gentle mechanical treatment steps, i.e. performed at low pressures, that induce cavitations in the outer cell wall without disrupting the cell completely, and making the inner cell wall accessible for enzymes in further process steps.

Before step a) is performed, fermentation media components such as glucose and minerals are preferably removed by a washing step. This might have an impact on the performance of the biotechnological process as extracellular and interstitial compounds are enriched.

The bead milling step step of process step a) is a well-known technique that has been used for a long time in industry in the ceramics, pharmaceutical, paint, paper and cosmetics industries and is now also used in biotechnology for the disruption of cells.

The bead milling system consists of a moving chamber filled with ceramic beads, which are usually less than 3 mm in size. The collisions between the beads damage the cell walls or the outer cell wall layer of the cells in between, causing cavitations.

Preferably, a water to biomass ratio of 4 and more or 10 or less is subjected to the process step of a).

Preferably, the bead milling step of process step a) is carried out at a rotational speed of between 2000 and 6000 RCF for between 20 seconds to 200 seconds to cavitate only the outer cell wall layer.

High-pressure-homogenization is a process that uses high pressure, thereby inducing cavitations in the biomass cells. This process can be fine-tuned to achieve a gentle and partial breakage of the cell wall through setting the flow rate, the pressure, and the number of passes a suspension undergoes the homogenization process.

Preferably, high-pressure homogenization is done at a pressure between 250 and 2000 bar and at a flow rate between 80 l/h and 80000 I/h, for a number of passes between 1 pass and 4 passes. More preferably, the mass of water to mass of biomass ratio should be 2 or more or 10 or less, preferably 4.

Cytolysis is the osmotic lysis of the cell membrane and the resulting release of the cell contents.

The benefits of cytolysis is that it is the most gentle method to disrupt the cells, whereby the cellular components are released to the environment in an intact native form.

Process step c) preferably serves to obtain lipids, proteins and vitamins.

In a preferred embodiment of the biotechnological process according to the first aspect, process step c) is carried out between 23°C and 60°C, more preferably process step c) is carried out at 45°C to 60°C for between one hour and 20 hours, more preferably between one hour and 6 hours, in order to increase the kinetic energy of the water and to increase the diffusion rate of the water into the cells.

Preferably, a water to biomass ratio of 5 and more or 20 or less is subjected to the process step of c).

Preferably, the mass of water to the mass of biomass in the suspension resulting from process step c) is adjusted in the range of 15 or more and 20 or less, and more preferably 15.

The distilled water diffuses into the interior of the microorganism cells. The cell absorbs water until it swells and the cell membrane breaks, releasing the cell contents. This process is known as osmotic lysis.

The bursting of the cell membrane is therefore due to the cellular osmotic equilibrium mechanism, which attempts to balance the concentration of solutes inside the cell with that outside the cell. The environment of the cell is therefore hypotonic, and water flows into the cell. The osmotic forces thus overcome the turgor (i.e. the internal cell pressure that normally stabilises the cell) and as a result the cell interior is released.

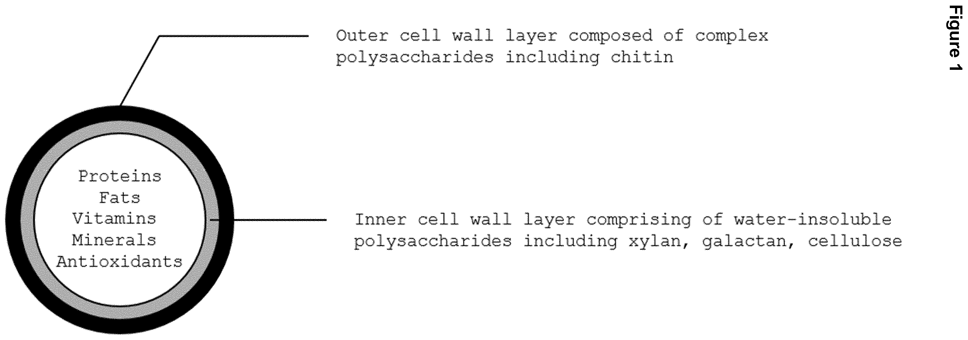

Figure 1 shows the cell wall structure of microalgae as an example of a cell wall-enclosing microorganism, particularly a cell wall structure of microalgae Chlorella vulgaris.

In a preferred embodiment, the biotechnological process comprises a) a process step selected from the group consisting of a bead milling step, a high-pressure homogenization step, an ultrasound treatment step or a combination thereof, b) an enzymatic treatment step and c) a cytolysis step.

In a preferred embodiment, the biotechnological process comprises a) a bead milling step and/or an alkaline treatment step, b) an enzymatic treatment step and c) a cytolysis step which takes place after process step a) and/or after process step b).

Preferably, the cell wall-embracing microorganisms are cultured prior to the process according to the invention and centrifuged at between 3000 and 6000 RCF, more preferably between 1000 RCF and 5000 RCF, at a rate between 100 l/h and 40000 l/h. The pellet is then subjected to the process according to the invention.

The starting material for the biotechnological process is preferably dry and/or wet microalgae biomass.

Microalgae are among the fastest growing organisms on earth. They can reproduce within hours and only need CO₂ and light to grow in fresh water, wastewater or seawater. An increased level of nutrients, such as those found in industrial process water, together with other growth-promoting compounds, can further increase the biomass growth of microalgae.

In case active biomass (autotrophic, heterotrophic and mixotrophic organisms) is processed, preferably the biomass is initially submitted to a concentration step, for example centrifugation, filtration, sedimentation or a combination thereof, after the cultivation process, and then inactivated by washing with water at a temperature between 50 °C and 75°C, and the resulting in inactivated biomass is subjected to a centrifugation step to remove residual fermentation media compounds, whereby the water can be de-ionized or normal water.

Preferably, washing is carried out in a heat-controlled continuously stirred tank reactor, mixing tank with heat control, or a bioreactor, whereby water is added to the biomass until a suspension with water to biomass ratio between 3 and 10 is obtained.

Preferably, washing takes place for a time period of 1 hour or less and 3 hours or more.

Preferably, centrifugation is performed with a disc centrifuge at a force between 1000 RCF and 5000 RCF and a rate between 100 l/h and 40000 I/h.

Preferably, the pellet of the centrifugation step of the inactivated biomass is subjected to a further washing step with de-ionized water to recover extra-cellular and interstitial compounds.

Preferably, in case an already inactive biomass is submitted to the process, the above described further washing step of the inactivated biomass is a first washing step of the inactive biomass.

In a preferred embodiment of the biotechnological process according to the first aspect, a centrifugation step is performed before the process step a), wherein a pellet and a supernatant is obtained.

Preferably, the centrifugation step is preceded by the washing step described above (second washing step of the inactivated biomass or first washing step of the inactive biomass), whereby the interstitial compounds and extracellular compounds are recovered in the supernatant. This stream is also labeled as Product A.

Preferably, the centrifugation step is performed using a disc-stack centrifuge at a force between 1000 RCF and 5000 RCF and a rate between 100 l/h and 40000 I/h.

In a preferred embodiment of the biotechnological process according to the first aspect, the pellet of the centrifugation step performed before the process step a) is subjected to step a).

Preferably, the pellet is diluted with water with a dilution factor between 3 and 10 prior to subjection to step a) to create a suspension.

In a preferred embodiment of the biotechnological process according to the first aspect, the supernatant of the centrifugation step performed before the process step a) comprises interstitial compounds and extracellular compounds.

Interstitial compounds diffuse from the cytoplasmic space of the cell towards the extra-cellular environment by effect of concentration gradients.

Extracellular compounds comprise exo-polysaccharides that could hypothetically form biofilms on the outer cell wall of the cell.

Interstitial compounds and extracellular compounds comprise carbohydrates, minerals, antioxidants, vitamins, and fibres.

In a preferred embodiment of the biotechnological process according to the first aspect, the supernatant of the centrifugation step performed before the process step a) is concentrated and/or dried.

Preferably, concentration takes place through an evaporation process at a pressure between 100 mbar and 500 mbar at a temperature between 80°C and 100 °C to remove excess water and to form a viscous syrup.

Preferably, the viscous syrup is further boiled under vacuum.

Preferably, a seed crystal is added to the viscous syrup at a concentration of 0.5 - 5% relative to the syrup solution by mass.

Preferably, crystallization takes place at a pressure between 100 mbar and 400 mbar at a temperature between 60°C - 80°C. More preferably, the temperature is lowered gradually into a temperature range between 45°C - 55°C.

Preferably, crystallization takes place for 1 - 3 hours.

Preferably, the formed crystals are separated by centrifugation at a force between 1000 RCF and 2000 RCF and a rate between 100 l/h and 40000 I/h.

Preferably, the crystals are dried using a rotary drum drier or a fluidized bed drier at a temperature between 60°C - 80°C.

In case concentration is performed alternatively or in additon, concentration preferably takes place through a nanofiltration process and/or reverse osmosis at a pore size of 0.1 nm to 1 nm.

Preferably, the resulting retentate is dried using common drying techniques such as spray drying, oven dying, freeze drying, rotary drum drying, fluidized bed drier or conveyor belt drying. More preferably, the resulting retentate is dried using rotary drum drying or a fluidized bed drier.

In a preferred embodiment of the biotechnological process according to the first aspect, the process step b) comprises addition of at least one enzyme selected from the group consisting of lysozyme, cellulase, xylanase, pectinase, hemicellulase, beta-glucanase, arabinase, beta-glucosidase or a combination thereof, wherein lysozyme or combination of lysozyme with cellulase, xylanase, pectinase, hemicellulase, beta-glucanase and/or arabinase is preferred.

The addition of lysozyme and/or an enzyme or combination of enzymes from the carbohydrases class allows the cell wall polysaccharides to hydrolyze and expose the cell gently.

In a further preferred embodiment of the biotechnological process according to the first aspect, the process step b) comprises the addition of lysozyme, wherein lysozyme is preferably added in a concentration between 0.05% and 1%.

Preferably, lysozyme is added at a dosage of 0.025% - 1% relative to biomass, and cellulase and xyalanase are added with a dosage of 0.025% relative to the biomass each.

In a further preferred embodiment of the biotechnological process according to the first aspect, the process step b) further comprises the addition of cellulase and/or xylanase, wherein cellulase is preferably added in a concentration between 0.025% - 1% and/or xylanase is preferably added in a concentration between 0.025% - 1%.

Preferably, enzymatic treatment takes place with an enzyme dosage of 0.025% - 1% of relative to the biomass, and a treatment time between 1 hour and 6 hours.

More preferably, in the case where Chlorella vulgaris is used as feedstock and the digestion of inner cell wall material is desired, an enzyme cocktail of xylanase, hemicellulase, beta-glucanase, and pectinase, is used at a temperature of 50 °C, a pH between 5 and 5.15, an enzyme dosage of 0.05%, and a treatment time of 2 hours.

In a further preferred embodiment of the biotechnological process according to the first aspect, process step b) is carried out at a pH between 3.8 and 6.7, for example between 4.5 and 5.5 or for example between 5.5 and 6.

Preferably, enzymatic treatment is carried out in a heat-controlled continuously stirred tank reactor, mixing tank with heat control, or a bioreactor, equipped with pH sensors.

Enzymatic treatment serves to digest inner and/ or outer cell wall material in a gentle manner, preserving the integrity of the cell, owing to the selectivity and mild nature of the enzymatic digestion process, and enabling the extracting of individual compounds with high purity.

Enzymatic treatment serves to target the polysaccharides making up the inner cell wall with the aim to weaken the cell wall and increase the permeability of the cell through reducing cell wall hydrophobic structures.

Enzymatic treatment further preserves the physio-chemical properties of the recovered compounds, which would be otherwise destroyed, if harsh conditions like high mechanical energy are used.

Also, valuable cell wall material is recovered, which include dietary fibers, proteins, and other valuable compounds.

Lysozyme is an enzyme that catalyses the breakage of the 1,4-beta bond between N-acetylglucosamine and N-acetylmuramine groups of the peptidoglycan polymer or chitin (which is composed of N-acetylglucosamine) of the outer cell wall layer, thus causing the outer cell wall layer to break down.

The enzyme cellulase catalyses the hydrolysis of cellulose of the inner cell wall layer to the monosaccharide glucose, thus leading to the dissolution of the inner cell wall layer.

The enzyme xylanase catalyses the hydrolysis of the hemicellulose xylan in the inner cell wall layer to the monosaccharide xylose, thus leading to the dissolution of the inner cell wall layer.

The enzymatic treatment with a combination of cellulase, xylanase, pectinase, hemicellulase, beta-glucanase, arabinase and/or beta-glucosidase with lysozyme leads to an increased rate of the entire cell wall being released compared to enzymatic treatment with lysozyme alone.

Enzymatic treatment with lysozyme and the combination of cellulase, xylanase, pectinase, hemicellulase, beta-glucanase, arabinase and/or beta-glucosidase with lysozyme does not lead to the dissolution of the cell membrane, as is the case with harsh treatment methods.

In a further preferred embodiment of the biotechnological process according to the first aspect, sugars are recovered in process step b).

Because the cell membrane is intact after process step b), the sugars obtained are not mixed with the proteins and lipids of the cell interior. These, in turn, can be selectively isolated in the subsequent process step c) without contamination with enzymes from process step b).

In a preferred embodiment of the biotechnological process according to the first aspect, a centrifugation step is carried out between process step a) and process step b) and the pellet obtained thereby is fed to process step b), more preferably a centrifugation step is carried out between process step a) and process step b) between 1000 RCF and 5000 RCF and a rate between 100 l/h and 40000 l/h and the pellet obtained in the process is fed to process step b).

Preferably, step a) is repeated after step b) for final cell disruption due to opening up the cell in a gentle way after the two cell walls have been compromised, and due to deactivating the enzymes.

Preferably, high-pressure homogenization is used when repeating step a) after step b), and more preferably high pressure homogenization is performed at a pressure between 250 and 2000 bar, at a flow rate between 80 l/h and 80000 I/h, for a number of passes between 1 and 4 passes.

In case a) is not repeated after step b), the biomass resulting as the pellet after centrifugation is optionally mixed with de-ionized water to induce cytolysis (step c)) as the final cell disruption step.

In a preferred embodiment of the biotechnological process according to the first aspect, a centrifugation step is performed after a) and/or b), wherein a pellet and a supernatant is obtained in each case.

Preferably, the centrifugation step is carried out after process step b) between 1000 RCF and 5000 RCF between 100 l/h and 40000 I/h, in which a pellet and supernatant are obtained.

Preferably, the centrifugation step is carried out a rate between 100 l/h and 40000 I/h.

In a preferred embodiment of the biotechnological process according to the first aspect, the supernatant of centrifugation step performed after a) and/or b) comprises sugars, oligosaccharides, fibers, vitamins, antioxidants, minerals and enzymes.

Preferably, the purified sugars comprise monosaccharides, more preferably the purified sugars comprise monosaccharides such as glucose, galactose, xylose, rhamnos, hemicellulose or a combination thereof.

The obtained supernant precisely is a stream, which is defined as Product B.

Preferably, enzymes are separated through ultrafiltration for 5 - 20 kDa.

Preferably, the ultrafiltration step is carried out at 5 KDa MWCO.

Preferably, the monosaccharides purified in the ultrafiltration step serve as a nutrient source for cell cultures.

The ultrafiltration step is also used to remove the enzymes of the enzymatic treatment step.

In a preferred embodiment of the biotechnological process according to the first aspect, the supernatant of centrifugation step performed after a) and/or b) is separated, concentrated and/or dried.

Preferably, concentration takes place through an evaporation process at a pressure between 100 mbar and 500 mbar at a temperature between 80°C and 100 °C.

Preferably, the concentrated and/or dried product comprising sugars, oligosaccharides, fibers, vitamins, antioxidants, minerals and enzymes is transferred into a heated stirred tank whereby the temperature is maintained between 50°C and 60°C to prevent fermentation.

Preferably, the concentrated and product is passed through a PES membrane of MWCO between 5 KDa and 20 KDa at 10 - 70 l/m²/h.

Preferably, the resulting pellet of the centrifugation step is diluted with water at a factor between 5 and 20, more preferably at a factor of 10, in a heat-controlled continuously stirred tank reactor, mixing tank with heat control, or a bioreactor. Even more preferably, the mass of water to mass of biomass should be 10. Mixing is carried out at a temperature between 18 °C and 60 °C for a mixing time between 1 hour and 6 hours.

In a preferred embodiment of the biotechnological process according to the first aspect, the obtained pellet is supplied to process step c).

In a preferred embodiment of the biotechnological process according to the first aspect, after process step c) at least one process step elected from the group consisting of an alkaline hydrolysis step, a centrifugation step, an extraction step, a separation step or a combination thereof is performed.

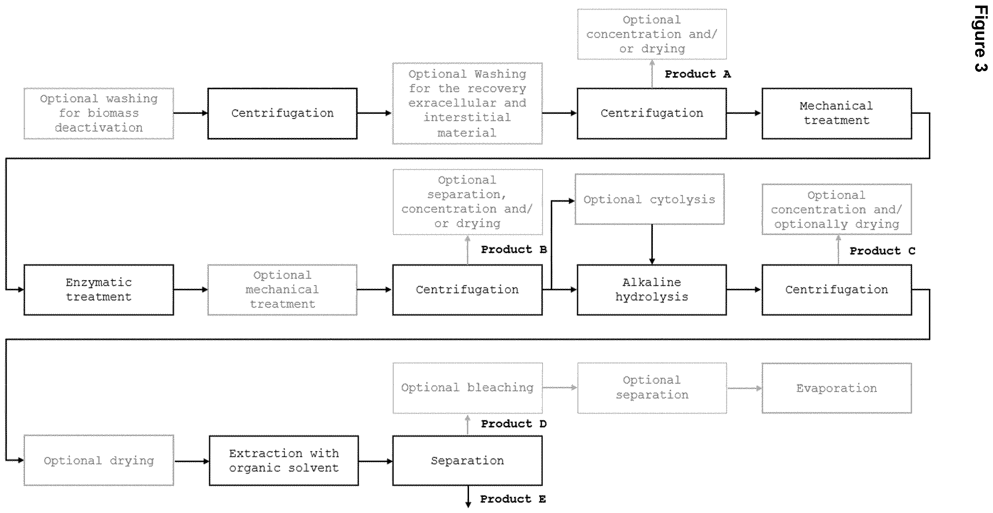

Figure 3 shows the process steps and their sequence. Microalgae are cited as an example of the starting material for microorganisms with a cell wall.

Alkaline agents such as hydroxide ions acting as nucleophiles attack the Carbonyl Carbon and lead to nucleophilic substation, resulting in proteins with smaller structures that are more readily soluble in water.

Preferably, a water to biomass ratio of 5 or more and 20 or less is subjected to the alkaline treatment step.

Preferably, heating is performed in the alkaline hydrolysis step at a temperature between 40°C and 65°C.

Preferably, alkaline hydrolysis takes place for a time period between 1 hour and 6 hours.

In a preferred embodiment of the biotechnological process according to the first aspect, the alkaline hydrolysis step is performed with sodium hydroxide as catalyst.

Preferably, sodium hydroxide is continuously added to maintain the pH in range between 7.5 and 9.5.

Preferably, after the alkaline hydrolysis step is followed by a centrifugation step.

Preferably, proteins are obtained in the supernatant of the centrifugation step, which is preceded by alkaline hydrolysis.

The liquid stream obtained in the supernatant of the centrifugation step, which is preceded by alkaline hydrolysis is labelled as Product C.

In a preferred embodiment of the biotechnological process according to the first aspect, an organic solvent is used in the extraction step.

Preferably, the extraction step is performed between 30 minutes and 3 hours.

Adding organic solvents to the pellet in the extractions step results in dissolving for example fats, oils and other organic solvent-soluble compounds.

Preferably, the organic solvent is added at a factor of 1 and more and 5 or less relative to the biomass by mass, more preferably at a factor of 3 relative to the biomass by mass.

Preferably, the temperature during adding the organic solvent is maintained between 20°C and 100 °C.

Preferably, one or more organic solvents selected from the group consisting of hexane, butane, propane, ethanol, or iso-propanol is used.

Preferably, organic solvents can be recovered after step b), in or after process step c).

Preferably, the separation step is performed using a temperature-controlled separator such as disc-stack centrifuge or three-phase separator at a force between 1000 RCF and 5000 RCF and a rate between 100 l/h and 40000 I/h, wherein the temperature is controlled to be between 10°C and 40°C.

The separation step yields a liquid fraction and a solid fraction. The liquid fraction contains the fats and/or oils, and other components which are labeled product D, and the solid fraction containing residuals which are labeled product E.

Preferably, in the separation step, a liquid fraction is separated by the solid fraction, wherein the solid fraction is discarded.

In a preferred embodiment of the biotechnological process according to the first aspect, after the separation step at least one process step elected from the group consisting of a bleaching step, an additional separation step, an evaporation step or a combination thereof is performed.

Preferably, the liquid fraction of the separation step undergoes the evaporation step.

Preferably, the evaporation step takes place at a temperature between 60 °C and 100 °C at a pressure between 100 mbar and 700 mbar.

Preferably, the components selected from the group consisting of fats and/or oils, carotenoids, vitamins or combinations thereof are concentrated by evaporating the organic solvent.

Preferably, the concentration is performed using a thin film evaporator where the solvent is evaporated at a pressure of 20 mbar and more or 400 mbar or less and a temperature between 70°C and 110°C.

In a further preferred embodiment of the biotechnological process according to the first aspect, step c) is followed by at least one process step selected from the group consisting of a microfiltration step, a centrifugation step, an ultrafiltration step, a spray drying step or a combination thereof.

Proteins are concentrated using ultrafiltration, preferably by using an ultrafiltration module running at 10 - 70 l/m²/h and a ceramic or PES membrane with a MWCO in the range of 5 - 300 KDa, the ultrafiltration module more preferably running at 30 l/m²/h, and the PES membrane has of MWCO 100 KDa.

The output is then dried using common drying techniques such as spray drying, oven dying, freeze drying, or drum drying, or conveyor belt drying.

Preferably, the ultrafiltration step is followed by a spray drying step to generate protein powder.

Preferably, spray drying at an inlet temperature between 150°C and 180°C and an outlet temperature between 60°C and 80°C, more preferably 70 and 75°C is performed.

Preferably, in the bleaching step bleaching earth clay at a percentage of 5%-10% relative to biomass is used.

Preferably, in the bleaching step the mixture is heated in a range between 70 - 120 °C.

Preferably, in the bleaching step stirring takes place for a time range between 30 minutes and 2 hours.

Preferably, a drying step of the pellet obtained by the centrifugation step is performed to partially remove water.

Preferably, the vitamins recovered include vitamin B, more preferably the vitamins recovered include B12.

The interstitial and extracellular compounds comprise carbohydrates, minerals such as iron and zinc, fibers, and vitamin B, for example vitamin B12.

Preferably, the proteins recovered include proteins in a native state.

Preferably, the drying step is performed using an industrial drying method, for example solar-drying, oven-drying, freeze drying or spray drying, and more preferably, in a freeze dryer for a temperature range between -20°C and -50°C, a pressure between 0.1 and 1 mbar, and a drying time between 24 and 72 hours.

In a preferred embodiment of the biotechnological process according to the first aspect, components selected from the group consisting of proteins, fats and/or oils, carotenoids, a sludge containing spent biomass, vitamins or combinations thereof are obtained from microorganisms of genus Chlorella, genus Nannochloropsis, genus Scenedesmus or genus Schizochytrium after step b), in or after process step c).

In a further preferred embodiment of the biotechnological process according to the first aspect, components selected from the group consisting of proteins, vitamins, fats or a combination thereof are obtained from microalgae of the genus Chlorella or the genus Nannochloropsis in process step c).

In another preferred embodiment of the biotechnological process according to the first aspect, the protein concentration is determined after at least one of the process steps mentioned in aspect 1.

**In a second aspect**, the invention provides for the biotechnological process according to the first aspect for use of the components obtained by the biotechnological process as nutrient composition, food product, food supplement, foodstuffs, food ingredients, pharmaceutical ingredients, feed ingredients, cosmetics, biofertilizer, soil additive or culture medium for cell cultures.

In a preferred embodiment, the biotechnological process according to the first aspect is for use of the components obtained by the process as a nutrient composition, food product, food supplement, foodstuffs, food ingredients, pharmaceutical ingredients or culture medium for cell cultures.

The combination of these process steps makes it possible to provide interstitial compounds, extracellular compounds, sugars, oligosaccharides, fibers, vitamins, antioxidants, minerals, enzymes, proteins, fats and/or oils, carotenoids, a sludge containing spent biomass, vitamins or combinations therof, in a native state, in high yields and specifically from microorganisms with a cell wall, in a gentle, efficient and inexpensive process, which can be used as a nutrient composition, food product, food supplement, foodstuffs, food ingredients, pharmaceutical ingredients or culture medium for cell cultures.

The present invention is further disclosed by the following non-limiting examples.

### EXAMPLES

### Example 1

### Microalgae cultivation

In this example, Chlorella vulgaris UTEX 395 is used, which is a well known strain and used as a standard. The culture is obtained in a 100 mL inoculum with a 250 mL Erlenmeyer flask with 1 mL F/2 medium per litre of fresh water standard according to the manufacturer's protocol (Algaeresearch Supply, San Diego, USA).

Cultivation is carried out according to the protocol described in Barros et al including several modifications described below. (Heterotrophy as a tool to overcome the long and costly autotrophic scale-up process for large scale production of microalgae (2019). Scientific Reports, 9(1), 13935).

Glucose is added to achieve a C:N ratio of 6.7 and the pH is adjusted to 6.5 by adding ammonia. After two days, the culture is transferred to a 5 L bioreactor (IKA, Germany), which is aerated at 1 vvm and stirred at 500 rpm. At the beginning of the cultivation, 1 ml/l F/2 medium is added, and the cultivation is carried out in a fed-batch process, with glucose being added at a rate of 15 g/l/h at the beginning of the exponential growth phase. Ammonia is added regularly to maintain the pH at 6.5. The harvested culture is centrifuged at 3000 rpm for 10 min, the supernatant is decanted and the pellet is dried at 70 °C for 2 h. The pellet is then resuspended in distilled water to achieve a final concentration of 200 g/l.

### Example 2: Extraction process with cytolysis

10 grams of dry Chlorella vulgaris biomass are suspended in 40 grams of de-ionized water to bring the biomass initial concentration in the solution to 250 g/L. The suspension is stirred for 1 hour at 350 rpm using a magnetic stirrer while the temperature is maintained at 50 °C. The suspension is centrifuged at 2000 RCF for 15 minutes and the supernatant is collected. The pellet is resuspended in 30 grams of de-ionized water and bead milled at 4000 rpm for 1 minute using Ultra-Turrax Tube Drive Control (IKA, Germany). The pH of the suspension is adjusted to 6.0 using Di-Potassium Hydrogen Phosphate and the suspension is transferred into a rotated incubator where Lysozyme was added at a dosage of 0.1% relative to the biomass by mass. Shaking takes place for 1 hour at 37°C. The pH is then brought to 5 using Citric Acid and Cellulase and Xylanase are added at 0.1% each relative to the biomass by mass. Shaking takes place for 1 hour at 50 °C. The sample is centrifuged at 2000 RCF for 30 minutes and the pellet is collected and resuspended in 60 grams of de-ionized water. The sample is transferred to a rotating incubator and shaking takes place at 50°C for 2 hours at 500 rpm. The sample is transferred to a flask and place on a magnetic stirrer where Sodium Hydroxide is added until the pH reaches 8.5 and the temperature is maintained at 50°C. Stirring takes place for 4 hours and the pH is continuously adjusted to 8.5 as it drops. The sample is centrifuged at 4000 RCF for 30 minutes at 4°C. The supernatant containing the protein is collected.

Protein content is determined using the Dumas apparatus. A nitrogen multiplication factor of 6.25 is used.

### Example 3: Extraction process without cytolysis with focus on the protein extraction

500 grams of dry Chlorella vulgaris biomass are suspended in 2000 grams of de-ionized water to bring the biomass initial concentration in the solution to 250 g/L. The suspension is stirred for 1 hour at 350 rpm using a magnetic stirrer while the temperature is maintained at 50 °C. The suspension is centrifuged at 2000 RCF for 15 minutes and the supernatant is collected. The pellet is resuspended in 500 grams of de-ionized water and the resulting solution is passed once through a Panda high-pressure homogenizer (GEA, Germany) operating at 1000 bar. The solution is transferred into a flask and placed on a magnetic stirrer. The pH is adjusted to 5 using Citric Acid and temperature is maintained at 50°C. Viscozyme L (Novozymes, Denmark) is added at 0.3% relative to the biomass by mass and mixing takes place at 300 rpm for 2 hours. The solution is then passed once through the high-pressure homogenizer operating at 1000 bar. The homogenized solution is centrifuged at 3000 RCF for 30 minutes at 12°C. The resulting pellet is resuspended in 4000 grams of de-ionized water and the pH is adjusted to 8.8 using Sodium Hydroxide. The solution is stirred at 350 rpm for 4 hours and the temperature is maintained at 50°C. The pH is continuously adjusted to 8.8 as it drops. The solution is then centrifuged at 4000 RCF for 45 minutes at 4°C. The supernatant containing the protein is collected.

### Example 4: Extraction process without cytolysis with a focus on oil extraction

100 grams of dry Chlorella vulgaris biomass are suspended in 300 grams of de-ionized water to bring the biomass initial concentration in the solution to 333.3 g/L. The solution is passed once through a Panda high-pressure homogenizer (GEA, Germany) operating at 1000 bar. The solution is transferred into a flask and placed on a magnetic stirrer. The pH is adjusted to 5 using Citric Acid and temperature is maintained at 50°C. Viscozyme L (Novozymes, Denmark) is added at 0.5% relative to the biomass by mass and mixing takes place at 300 rpm for 2 hours. The solution is centrifuged at 3000 RCF for 30 minutes at 12°C. The resulting pellet is resuspended in 200 grams of Ethanol and stirred at 300 rpm for 2 hours and stirred in an extraction flask equipped with a cooling column. The temperature is maintained at 75°C. The solution is cooled down and then centrifuged at 4000 RCF for 45 minutes at 40°C. The supernatant containing the solvent, oils, and carotenoids is collected.

### Example 5: Product A concentration

Product A contains water, interstitial compounds, and extracellular compounds resulting as the supernatant in the centrifugation process between and the second optional washing and the first gentle mechanical treatment step.

500 ml of Product A are transferred to a rotary evaporator operating at 280 mbar. The temperature is maintained at 90°C. Water evaporation takes place until the solution is concentrated 10-fold. A thick syrup is formed and is further boiled at 280 mbar. A seed crystal from previous trials is added at a concentration of 0.5% relative to the syrup solution by mass. Crystallization takes place at the set pressure of 280 mbar. The temperature is lowered gradually into a temperature range between 55°C and boiling takes place for 1 hour. The solution is then centrifuged at 1000 RCF for 30 minutes at 10°C. The pellet containing the crystals is collected and dried in a vacuum drier at 40°C.

**Table 1: Macro- and micronutrient composition of Product A after concentration and drying**

| Substance | Unit | Result | Method |
|---|---|---|---|
| Total dietary fiber | g/100 g | 5.35 | §64 LFGB L 00.00-18 : 2017-10 |
| Iron (Fe) | mg/ Kg | 163 | DIN EN 16943 : 2017-07 |
| Zinc (Zn) | mg/ Kg | 368 | DIN EN 16943 : 2017-07 |
| Vitamin B12 | µg/100 | 50.1 | Journal of AOAC Vol.91 No4:2008(1B) |
| | RCF | | |

### Example 6: Product C concentration

Product C is liquid stream containing water, proteins, and other compounds, resulting from the centrifugation after the Alkaline hydrolysis step.

4000 ml of Product C are placed in flask and stirred gently. Hydrochloric acid 7% is added until the pH is brought to 4.5. The solution is stored at 4°C for 1 hour until enough aggregation takes place. The solution is then centrifuged at 3000 RCF for 30 minutes at 4°C and the pellet containing the protein is collected and diluted with 2000 mL of de-ionized water. The pH is brought to 7.5 by adding Di-Potassium Hydrogen Phosphate. The solution is stirred at 50°C to prevent fermentation and pumped through a PES membrane to remove excess salt and residuals until the solution is concentrated by 10-fold. The concentrate containing the protein is collected and spray dried using a Büchi Spray Dryer B-290 (Büchi, Switzerland) at an inlet temperature of 160°C and an outlet temperature of 70°C. The resulting protein powder is collected.

### Example 7: Product D concentration

Product D is liquid or semi-solid stream containing fats and/or oils, carotenoids, and other compounds, resulting from the separation step after organic-solvent extraction step.

500 ml of product D are placed in an extraction flask equipped with a cooling column. 20 grams of Activated Bleaching Earth and 2 grams of Activated Carbon are added to the solution. Stirring takes place for 1 hour at 75°C. The solution is then cooled down and centrifuged at 2000 RCF for 30 minutes at 40°C. The supernatant containing the oil is collected and transferred to a rotary evaporator where the pressure is maintained at 400 mbar. Initially, the temperature is set at 70°C and the solvent is evaporated for 1 hour. The pressure is then set at 300 mbar and temperature is increased to 90°C, the water is evaporated for 30 minutes. The solution containing semi-refined oil is collected.

**Table 2: Fatty acid composition of Product D after concentration**

| Substance | Unit | Result | Method |
|---|---|---|---|
| Palmitic acid (C 16:0) | % | 27.9 | DGF C-VI 11a : 2016 (mod.) + DGF C-VI 10a : 2016 (mod.) |
| Palmitoleinic acid (C 16: 1) | % | 1.2 | DGF C-VI 11a : 2016 (mod.) + DGF C-VI 10a : 2016 (mod.) |
| Hexadecadioic acid C16:2 (n-4) | % | <0.1 | DGF C-VI 11a : 2016 (mod.) + DGF C-VI 10a : 2016 (mod.) |
| Hexadecatrienic acid C16:3 omega-3 | % | 8.9 | DGF C-VI 11a : 2016 (mod.) + DGF C-VI 10a : 2016 (mod.) |
| Stearic acid (C 18:0) | % | 0.5 | DGF C-VI 11a : 2016 (mod.) + DGF C-VI 10a : 2016 (mod.) |
| Octadecenoic acid trans-isomers C 18:1 trans | % | <0.2 | DGF C-VI 11a : 2016 (mod.) + DGF C-VI 10a : 2016 (mod.) |
| Oleic acid C 18:1 | % | 19.6 | DGF C-VI 11a : 2016 (mod.) + DGF C-VI 10a : 2016 (mod.) |
| cis-vaccenic acid C 18:1 | % | 0.5 | DGF C-VI 11a : 2016 (mod.) + DGF C-VI 10a : 2016 (mod.) |
| Octadecadienoic acid trans-isomers C 18:2 trans | % | <0.3 | DGF C-VI 11a : 2016 (mod.) + DGF C-VI 10a : 2016 (mod.) |
| Linoleic acid C 18:2 omega-6 | % | 28.5 | DGF C-VI 11a : 2016 (mod.) + DGF C-VI 10a : 2016 (mod.) |
| Octadecatetrienic acid, transisomers C 18:3 trans | % | <0.4 | DGF C-VI 11a : 2016 (mod.) + DGF C-VI 10a : 2016 (mod.) |
| alpha-linolenic acid C 18:3 omega-3 | % | 12.2 | DGF C-VI 11a : 2016 (mod.) + DGF C-VI 10a : 2016 (mod.) |
| Eicosenoic acid C 20:1 | % | <0.2 | DGF C-VI 11a : 2016 (mod.) + DGF C-VI 10a : 2016 (mod.) |
| Sum Saturated Fatty Acids | % | 28.4 | Calculated |
| Sum Mono-Unsaturated Fatty Acids | % | 21.3 | Calculated |
| Sum Poly-Unsaturated Fatty Acids | % | 49.6 | Calculated |
| Sum Trans Fatty Acids | % | <0.1 | Calculated |

### Brief description of the drawings

Figure 1 is a diagram of the outer cell wall layer, the inner cell wall layer and intercellular components of microorganisms with a cell wall.

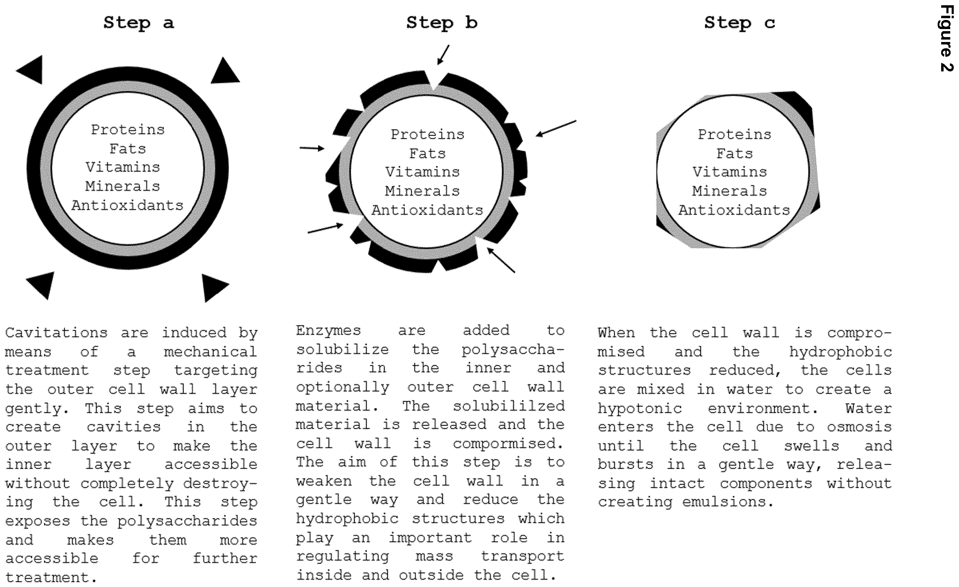

Figure 2 is a diagram providing an overview of process steps a), b) and c).
Figure 3 is a schematical overview of the process steps and their sequence.

Product A contains water, interstitial compounds, and extracellular compounds resulting as the supernatant in the centrifugation process between and the second optional washing and the first gentle mechanical treatment step.

Product B is liquid stream containing water, enzymes, sugars, oligosaccharides, and other compounds resulting from the centrifugation in the centrifugation process between the enzymatic step or the second gentle mechanical treatment step, and the alkaline hydrolysis step.

Product C is liquid stream containing water, proteins, and other compounds, resulting from the centrifugation after the alkaline hydrolysis step.

Product D is liquid or semi-solid stream containing fats and/or oils, carotenoids, and other compounds, resulting from the separation step after organic-solvent extraction step.

Product E is sludge containing spent biomass and water, resulting from the separation step after the organic-solvent extraction step.

## Claims

1. Biotechnological process for obtaining components from microorganisms with a cell wall, comprising the following process steps:
a) a process step selected from the group consisting of a bead milling step, a high-pressure homogenization step, an ultrasound treatment step or a combination thereof;
and
b) an enzymatic treatment step;
and optionally
c) a cytolysis step.

2. Biotechnological process according to claim 1, wherein a centrifugation step is performed before the process step a), wherein a pellet and a supernatant is obtained.

3. Biotechnological process according to claim 2, wherein the pellet is subjected to step a).

4. Biotechnological process according to claim 2, wherein the supernatant is concentrated and/or dried.

5. Biotechnological process according to any one of claims 1 to 4, wherein the process step b) comprises addition of at least one enzyme selected from the group consisting of Lysozyme, Cellulase, Xylanase, Pectinase, Hemicellulase, Beta-Glucanase, Arabinase, Beta-Glucosidase or a combination thereof, preferably the process step b) comprises addition of Lysozyme.

6. Biotechnological process according to any one of claims 1 to 5, wherein a centrifugation step is performed after a) and/or b), wherein a pellet and a supernatant is obtained in each case.

7. Biotechnological process according to claim 6, wherein the supernatant is separated, concentrated and/or dried.

8. Biotechnological process according to any one of claims 1 to 7, wherein the biotechnological process comprises step c) and after process step c) at least one process step elected from the group consisting of an alkaline hydrolysis step, a centrifugation step, an extraction step, a separation step or a combination thereof is performed.

9. Biotechnological process according to claim 8, wherein the alkaline hydrolysis step is performed with sodium hydroxide as catalyst.

10. Biotechnological process according to claim 8 or 9, wherein an organic solvent is used in the extraction step.

11. Biotechnological process according to any one of claims 8 to 10, wherein after the separation step at least one process step elected from the group consisting of a bleaching step, an additional separation step, an evaporation step or a combination thereof is performed.

12. Biotechnological process according to claim 2 or 4, wherein the supernatant comprises interstitial compounds and extracellular compounds.

13. Biotechnological process according to claim 6 or 7, wherein the supernatant comprises sugars, oligosaccharides, fibers, vitamins, antioxidants, minerals and enzymes.

14. Biotechnological process according to any one of claims 1 to 13, wherein components selected from the group consisting of proteins, fats and/or oils, carotenoids, a sludge containing spent biomass, vitamins or combinations thereof are obtained from microorganisms of genus Chlorella, genus Nannochloropsis, genus Scenedesmus or genus Schizochytrium after step b), in or after process step c).

15. Use of the components obtained by the biotechnological process according to any one of claims 12 to 14 as a nutrient composition, food product, food supplement, foodstuffs, food ingredients, pharmaceutical ingredients, feed ingredients, cosmetics, biofertilizer, soil additive or culture medium for cell cultures.
